# EUROPEAN PATENT SPECIFICATION

(11) **EP 3 880 170 B1**
(45) Date of publication and mention of the grant of the patent: **31.12.2025**
(21) Application number: 19809918.6
(22) Date of filing: 12.11.2019
(51) Int. Cl.: A61K 9/00, A61K 47/46

(54) **COMPOSITION CONTAINING CINNAMON PHYTO COMPLEX TO BE USED AS A COADJUVANT IN THE TREATMENT OF DI OF THE RESPIRATORY TRACT AND OF COLD IN GENERAL**
ZUSAMMENSETZUNG MIT ZIMT-PHYTO-KOMPLEX ZUR VERWENDUNG ALS CO-ADJUVANS IN DER BEHANDLUNG DER ATEMWEGE UND VON ERKÄLTUNGEN IM ALLGEMEINEN
COMPOSITION CONTENANT UN PHYTOCOMPLEXE DE CANNELLE À UTILISER EN TANT QUE COADJUVANT DANS LE TRAITEMENT DU DL DU TRACTUS RESPIRATOIRE ET D'UN REFROIDISSEMENT EN GÉNÉRAL

(30) Priority: 12.11.2018 IT 201800010241
(43) Date of publication of application: 22.09.2021
(73) Proprietor: Cinnapharm S.A.S. di Tombolato Alberto Denis e C., 31030 Castello di Godego (TV) (IT)
(72) Inventor: TOMBOLATO, Alberto Denis, 31030 Castello Di Godego (TV) (IT)
(74) Representative: Rocchetto, Elena
(86) International application number: PCT/IB2019/059689
(87) International publication number: WO 2020/100017

(56) References cited:
- EP-B1- 2 303 301
- CN-A- 107 847 426
- US-B1- 7 150 888
- KAMILLA ÁCS ET AL: "Antibacterial activity evaluation of selected essential oils in liquid and vapor phase on respiratory tract pathogens", BMC COMPLEMENTARY AND ALTERNATIVE MEDICINE, BIOMED CENTRAL LTD, LONDON, UK, vol. 18, no. 1, 27 July 2018 (2018-07-27), pages 1 - 9, XP021258911, DOI: 10.1186/S12906-018-2291-9
- A. FABIO ET AL: "Screening of the antibacterial effects of a variety of essential oils on microorganisms responsible for respiratory infections", PHYTOTHERAPY RESEARCH., vol. 21, no. 4, 26 February 2007 (2007-02-26), GB, pages 374 - 377, XP055592577, ISSN: 0951-418X, DOI: 10.1002/ptr.1968
- INOUYE S ET AL: "SCREENING OF THE ANTIBACTERIAL EFFECTS OF A VARIETY OF ESSENTIAL OILS ON RESPIRATORY TRACT PATHOGENS, USING A MODIFIED DILUTION ASSAY METHOD", JOURNAL OF INFECTION AND CHEMOTHERAPY, CHURCHILL LIVINGSTONE, TOKYO, JP, vol. 7, no. 4, 1 January 2001 (2001-01-01), pages 251 - 254, XP001146833, ISSN: 1341-321X, DOI: 10.1007/S101560170022

## Description

The present invention concerns the compositions containing cinnamon phyto complex, in the form of essential oil and/or essence, for the treatment of diseases of the respiratory tract and of colds in general.

Infectious diseases are among the most treated diseases in terms of public health and, in particular, they generally are seasonal infections affecting the respiratory tract.

The sometimes unjustified, excessive and unnecessary use of antibiotics for the treatment of these diseases has had negative repercussions on the phenomenon known as drug resistance, thus making it more difficult to treat respiratory diseases, especially in the patient categories at risk, like children, older people, patients suffering from heart diseases and from diabetes etc.

The present patent is related to the US patent US 7150888, which exclusively concerns the prevention of respiratory diseases through the use of two active principles, that is, eucalyptus essential oil and tea tree oil.

According to the method illustrated in the document cited above, the vapours of the essential oils specified above must be periodically and repeatedly inhaled. The document teaches that the inhalation must take place repeatedly at precise time intervals not exceeding 2 hours, based on the principle according to which these vapours, once inhaled, form a thin "anti-pathogenic" layer in the respiratory tract, protecting it from infections and substantially preventing microorganisms from settling and proliferating in the patient's organism. The document thus limits the use of vapours of essential oils to prophylactic treatments capable of preventing an infection but only for a determined period of time after the inhalation.

In the inhalation of vapours, the size of the particles of essential oil is generally included between 20 and 180 µm and therefore these particles reach only the upper respiratory tract, creating the above mentioned protective film which serves to stop any microorganisms which may get into the patient's organism.

This method, as explicitly specified in the document, has only a preventive purpose but cannot be effectively employed in the case where a disease of the respiratory tract is already affecting the patient. In this case, in fact, the repeated inhalation of essential oils, as suggested in the document, would create a film on the surface with which they come into contact, with no effect on the disease already in progress. No part of the document teaches that the inhalation of essential oils can be used in any way as a coadjuvant in the treatment of diseases of the respiratory tract already in progress, and the document does not suggest that essential oils should be administered using methods different from the inhalation of their vapours, either.

Among the countless essential oils suited to be used for the creation of said protective film only for preventive and prophylactic purposes but not for therapeutic purposes, the document mentions also the essential oil of *Cinnamomum camphora* in the quantity of 5% in volume, in addition to other essential oils, among which 50 % of *Eucalyptus globulus* and 25% of *Melaleuca alternifolia*, where the last two components clearly serve the main function.

Cinnapharm's patent application concerns A composition suited to be nebulised through aerosol therapy and to be used as a coadjuvant in the treatment of colds and diseases of the respiratory tract in general, comprising cinnamon-based phytocomplexes characterized in that it comprises:
- water;
- copper gluconate;
- polysorbate 80;
- cinnamon essential oil;
- thyme essential oil;
- grindelia fluid extract;
- erysimum fluid extract;
- glycerine macerate of blackcurrant (ribes nigrum) and a further ingredient selected between eucalyptus essential oil and mountain pine essential oil.

The composition that is the subject of the present invention does not include pharmacological active ingredients.

One of the main tasks of the present invention is to provide a composition that can be used as a coadjuvant in the treatment of respiratory diseases already affecting the patient and contains natural ingredients, thus avoiding or reducing the use of pharmacological therapies.

The present invention can thus be effectively used in therapeutic treatments, meaning treatments intended to resolve respiratory diseases already in progress, wherein the composition that is the subject of the present invention is administered in nebulized form through aerosol therapy, for example by means of common aerosol therapy devices.

The present invention thus concerns a composition suited to be nebulized through aerosol therapy and to be used as a coadjuvant in the treatment of diseases of the respiratory tract in general, such as cold or influenza, already affecting the patient.

Differently from the administration through an atomizer or through vapour, with particle size generally ranging between 20 and 180 µm, nebulization through aerosol therapy guarantees that the size of the inhaled particles generally does not exceed 5 µm.

The effectiveness of the inhalation therapy and its ability to reach the upper and lower respiratory tract mainly depend on the size of the emitted particles. Actually, the atomization of a solution makes it possible to reach the upper respiratory tract, while the administration through nebulization, as proposed in the present patent, makes it possible to reach also the lower respiratory tract, thus providing a coadjuvant which is more specific for the treatment of respiratory diseases and colds in general.

The present invention is mainly focused on the use of cinnamon phyto complexes, in its variety *Cinnamomum zeylanicum*, in the solution for aerosol therapy. This medicinal plant has been selected following research conducted on the antimicrobial and antiviral properties of its extract.

The use of cinnamon, as extract, essence and aroma, represents the innovative feature of the preparation owing to the characteristics of the phyto complex of which it is constituted. This phyto complex, both alone and associated with other medicinal plants and oligoelements, is useful for improving the conditions of patients suffering from respiratory diseases, often related to the season, and favours their healing processes.

The composition that is the subject of the invention is a solution suited to be nebulized through aerosol therapy and is particularly recommended because of its direct action on the respiratory tract.

In its preferred form, said composition comprises cinnamon essential oil, in solution alone or with other plant extracts and/or oligoelements, to be nebulized and administered through aerosol therapy.

Cinnamon essential oil has an important antibacterial and antiviral action as a coadjuvant for the treatment of respiratory diseases, wherein said action proves to be surprisingly effective, according to the teachings of the present patent, when the composition is administered in nebulized form through aerosol therapy.

This composition may include a specific combination with other active principles, intended to create a synergistic effect to maximize the effectiveness of the treatment so as to properly moisten the mucosa and actually favour the production of fluid mucus and the natural drainage of secretions, with excellent results and in a relatively short time.

The inhalation of particles in the form of aerosol is to be considered useful for the correct functionality of the respiratory tract and, furthermore, generally has no side effects [1]. The composition disclosed herein comprises *Cinnamomum zeylanicum*, or cinnamon, known for its extended antimicrobial characteristics against some bacteria, both Gram-positive and Gram-negative, and on some viral strains.

Cinnamon is used in this composition as a fundamental active principle to achieve the desired effect, that is, to help and accelerate the cure of respiratory diseases already affecting the patient.

Several properties of cinnamon and, in particular, of its essential oil are currently recognized. Antiviral and antimicrobial properties stand out among them. For example, its antiviral activity has been described in a study published in the well-known magazine "Science", which was intended to describe the activity of cinnamaldehyde against adenovirus, an etiological agent that can cause infections in humans [28].

According to the most recent literature, the antiviral action mechanism of cinnamon consists in its inhibitory capacity against caspases and neuraminidases. Caspases are protease enzymes which are present in the cells in their inactive form and, following an apoptotic stimulus, are capable of generating a proteolytic cascade. According to the study mentioned above, the level of apoptosis in the groups treated with cinnamaldehyde was lower than that observed in the virus control groups, which suggested that the anti-ADV3 effect could be related to the apoptotic mechanism. The effect can be observed also on the surface of the ADV which, after the treatment with cinnamaldehyde, is not regular and globular any longer but rather morphologically reduced and irregular. Consequently, the virus capsid deteriorates, thus preventing the replication of the virus [28].

A second study concerns neuraminidases, enzymes which in type A viruses allow the exit of the viral particles from the host cell and thus facilitate the spread of the infection. The preferred drugs acting on neuraminidases are oseltamivir and zanamivir. And it is precisely with respect to the latter that the antiviral activity of Cinnamomum zeylanicum has been proven [29].

The antibacterial action mechanism is due to the inhibition of the formation of the biofilm of the bacterium itself. Cinnamaldehyde thus causes damage to the cytoplasmatic membrane and lysis in the cell wall, with a consequent loss of the cytoplasmatic contents. Scientific evidence makes it possible to state that cinnamon, thanks to its phyto complex, can be considered a new antimicrobial agent and can be used even alone or in any case in combination with the synthetic antibiotics available on the market.

A scientific study has proven the antibacterial properties of cinnamon against four Gram-negative bacteria (Escherichia coli, Klebsiella pneumoniae, Pseudomonas aeruginosa, Proteus vulgaris) and two Gram-positive bacteria (Bacillus subtilis e Staphylococcus aureus) [2]. These characteristics are dependent on concentration [3].

Another important clinical study has shown an increase in the antimicrobial activity against Streptococcus aureus when certain antibiotics and cinnamon are administered together [4].

Other studies have also been conducted, which are aimed at identifying new strategies to increase the effectiveness of antibiotics while at the same time reducing the resistance of the bacteria. Literature on the subject confirms the synergistic action of piperacillin and cinnamon essential oil, which strengthens the antibacterial action of the treatment [5].

A similar result has been obtained also by another recent study which concerns the association of essential oils, including *Cinnamomum zeylanicum*, *Syzygium aromaticum L.*, *Zingiber cassumunar L.* and *Curcuma longa L.* essential oil, alone and combined with antibiotics, for example piperacillin, ciprofloxacin, imipenem and meropenem. The result showed the excellent antibacterial action of cinnamon essential oil, alone and associated with other agents, against Gram-negative bacteria [6].

It has also been stated that trans-cinnamaldehyde gives good antimicrobial results, especially against microorganisms transmitted through the air [7].

An important result has been highlighted also in regard to the effects of trans-cinnamaldehyde against the influenza virus A/PR/8, in vitro and in vivo.

All the above provides further evidence of the fact that *Cinnamomum zeylanicum* can be advantageously used to treat the diseases that affect the respiratory tract [8].

In the composition that is the subject of the present invention, antimicrobial properties are increased owing to the synergistic effect produced by the combination of cinnamon and *Thymus vulgaris* or thyme. The main constituents of this plant include thymol and carvacrol, which induce antiseptic, expectorant, mucolytic, antitussive and spasmolytic activities. This plant, in fact, is presently used in the treatment of diseases of the respiratory tract, and is useful when treating chronic and acute bronchitis, pertussis (whooping cough), asthmatic cough and catarrh. These properties have been recognized also by Commission E of the Bundesinstitut für Arzneimittel und Medizinprodukte (BfArM), the German regulatory authority [9].

Thyme has biocidal properties owing to the perturbation and destabilization it causes at the level of the bacterial membrane and to its interference with the intracellular sites of the same [10] [11].

Other important results have been obtained by another clinical study which showed the antimicrobial properties of the flavonoid fraction present in *Thymus vulgaris.* The use of this medicinal plant is supported by a scientific rationale also in the case of inflammation and infections affecting the upper respiratory tract [12].

In the composition that is the subject of the present invention, in the formulation for adults and not in the paediatric formulation, the expectorant and antiseptic properties are further enhanced owing to the synergistic effect produced by the combination of cinnamon and essential oil of *Eucalyptus globulus* or eucalyptus.

Eucalyptus extract is traditionally used to treat acute bronchial diseases, colds, pharyngitis, sinusitis. The action mechanism of the essential oil of eucalyptus is linked to its ability to interfere with the formation of the biofilm and with the mature biofilm, in particular in the case of Staphylococcus aureus and Pseudomonas aeruginosa, which are often involved in antimicrobial resistance mechanisms [13].

Eucalyptus essential oil and its main constituent, 1,8-cineole, have antimicrobial properties against many bacteria, including Mycobacterium tuberculosis and methicillin resistant Staphylococcus aureus; it has also an antioxidant, anti-inflammatory and immune-stimulating action.

The inhalation of eucalyptus extract vapours is beneficial in the case of catarrhal diseases of the respiratory tract, asthma and chronic obstructive pulmonary disease (COPD) [14]. It has been shown that also long-term treatments with 1,8-cineole are beneficial and help prevent the occurrence of COPD and increase asthma control [15].

Regarding the capacity to increase innate cell-mediated immune response, it has been confirmed that it would be useful to study the oil extracted from different species of *Eucalyptus* to develop new classes of immune regulating substances that could serve as adjuvants in immunosuppressive and infectious diseases also in oncological patients [16]. In the paediatric formulation, from which eucalyptus essential oil has been excluded, the composition of the invention comprises mountain pine, which is useful for its balsamic bactericidal, anti-inflammatory and expectorant characteristics that help clear the upper and lower respiratory tract of catarrh [17].

The composition that is the subject of the invention comprises also *Grindelia robusta*, which is beneficial against bronchial diseases. Commission E of BfArM recommends its use in case of presence of catarrh in the upper respiratory tract [18], colds, asthma, bronchitis and pertussis [19].

Furthermore, given its emollient action, it can be used at the level of the oropharyngeal cavity, improving the tone of the voice [20]. The phenolic acids that constitute the phyto complex are responsible for the anti-inflammatory, antibacterial, spasmolytic and antioxidant effects of the plant [21].

Finally, the extract of Grindelia seems to be very effective in the treatment of inflammation of the oropharyngeal cavity, thanks to its capacity to reduce the accumulation of chemical mediators of the inflammation, like IL-6, TNF-α and some metalloproteinases [22].

The composition of the invention comprises also *Sisymbrium officinalis* or erysimum, which as an anti-inflammatory effect on the throat and on the upper respiratory tract following laryngitis, pharyngitis and tracheitis.

Clinical studies have focused on its antibacterial properties against five Gram-positive bacteria, nine ampicillin-resistant Gram-negative bacteria and four fungi [23]. *Sisymbrium officinalis*, or erysimum, also called "the Singer's Plant", showed a good ability to reduce the levels of histamine and leukotrienes, which are the chemical mediators of the inflammation and are involved in particular in asthma and bronchitis.

Its strong antimutagenic profile, furthermore, suggests that in-depth studies should be carried out in order to better assess its chemopreventive activity [24].

The composition of the invention comprises also Ribes nigrum (blackcurrant), rich in polyphenols which have an anti-inflammatory effect and lead to the inhibition of cPLA2α. Actually, it reduces the recruitment of leukocytes and the production of histamine, reduces the presence of mast cells in the inflamed area and has antioxidant properties [25]. Ribes nigrum also intervenes in the modulation of the inflammation that takes place in the respiratory tract in the presence of allergies, ensuring benefits in terms of control of the inflammation affecting the respiratory tract [26].

The composition of the invention comprises also copper gluconate, which is recognised as an important element for the biochemistry of the organism. Copper is currently used in infections because it intervenes in the normal functionality of the immune system; it intervenes in the mechanisms of cell catalysis, participating in the reactions of superoxide dismutase (SOD) and serving the function of an antioxidant, which is fundamental at cellular level to face the oxidative stress to which organisms are constantly exposed. Copper gluconate is effectively used also in the treatment of subjects exposed to cigarette smoke and environmental pollution. In oligotherapy, copper is also used as anti-infective, anti-inflammatory and immunostimulant agent, used in case of fever and in inflammatory and infectious processes, especially in the respiratory tract [27].

The preferred composition, in the formulation for adults, comprises:

| | |
|---|---|
| - sea water | 0.001-99.999 % |
| - copper gluconate | 0.001-0.1 % |
| - polysorbate 80 | 0.1-0.5 % |
| - cinnamon essential oil | 0.001-0.1 % |
| - thyme essential oil | 0.001-0.1 % |
| - eucalyptus essential oil | 0.001-0.1 % |
| - grindelia fluid extract | 0.001-0.1 % |
| - erysimum fluid extract | 0.001-0.1 % |
| - glycerine macerate of blackcurrant (ribes nigrum) | 0.001-0.1% |

The preferred composition, in the formulation for children, comprises:

| | |
|---|---|
| - sea water | 0.001-99,999 % |
| - copper gluconate | 0.001-0.1 % |
| - polysorbate 80 | 0.1-0.5 % |
| - cinnamon essential oil | 0.001-0.1 % |
| - thyme essential oil | 0.001-0.1 % |
| - mountain pine essential oil | 0.001-0.1 % |
| - grindelia fluid extract | 0.001-0.1 % |
| - erysimum fluid extract | 0.001-0.1 % |
| - glycerine macerate of blackcurrant (ribes nigrum) | 0.001-0.1 %. |

The formulations specified above, within the considered ranges, are especially effective when used as coadjuvants in the treatment of colds and respiratory diseases. They both come in a solution to be nebulized and are administered through aerosol therapy.

Therefore, with reference to the above description, the following claims are expressed.

### Bibliography

[1] Masakazu Umezawa et al. Effect of aerosol particles generated by ultrasonic humidifiers on the lung in mouse. Particle and Fibre Toxicology. Dec 2013; 10:64.
[2] Seenivasan Prabuseenivasan. In vitro antibacterial activity of some plant essential oils. BMC Complementary and Alternative Medicine. Nov 2006
[3] P. Goñi et al. Antimicrobial activity in the vapour phase of a combination of cinnamon and clove essential oils. Food Chemistry. Volume 116, Issue 4,15 October 2009, Pages 982-989
[4] J. Mahadlek et al., Combination Effects of the Antimicrobial Agents and Cinnamon Oil, Advanced Materials Research, Vol. 506, pp. 246-249, 2012
[5] Polly Soo Xi Yap, et al. Antibacterial Mode of Action of Cinnamomum verum Bark Essential Oil, Alone and in Combination with Piperacillin, Against a MultiDrug-Resistant Escherichia coli Strain. Journal of Microbiological Biotechnologies. 2015; 25(8), 1299-1306.
[6] Itsaraporn Utchariyakiat, et al. Efficacy of cinnamon bark oil and cinnamaldehyde on anti-multidrug resistant Pseudomonas aeruginosa and the synergistic effects in combination with other antimicrobial agents. BMC Complement Alternative Medicine. 2016; 16: 158.
[7] Kei Sato, et al. Antimicrobial Effect of trans-Cinnamaldehyde, (-)-Perillaldehyde, (-)-Citronellal, Citral, Eugenol and Carvacrol on Airborne Microbes Using an Airwasher. Biol. Pharm. Bull. 2006. 29(11) 2292-2294.
[8] Hayashi K et al. Inhibitory effect of cinnamaldehyde, derived from Cinnamomi cortex, on the growth of influenza A/PR/8 virus in vitro and in vivo. Antiviral Research. 2007 Apr;74(1):1-8.
[9] E.Campanini. Dizionario di fitoterapia e piante medicinali. Noviglio (MI). Tecniche Nuove. 2014.
[10] M. Cristani, M. D'Arrigo, G. Mandalari et al., Interaction of four monoterpenes contained in essential oils with model membranes: implications for their antibacterial activity. Journal of Agricultural and Food Chemistry, vol. 55, no. 15, pp. 6300-6308, 2007.
[11] A. Ultee, E. P.W. Kets, and E. J. Smid. Mechanisms of action of carvacrol on the food-borne pathogen Bacillus cereus. Applied and Environmental Microbiology. vol. 65, no. 10, pp. 4606-4610, 1999.
[12] Arifa Mehreen. Phytochemical, Antimicrobial, and Toxicological Evaluation of Traditional Herbs Used to Treat Sore Throat. Hindawi Publishing Corporation BioMed Research International. Apr 2016.
[13]Camporese A. In vitro activity of Eucalyptus and Juniperus communis essential oils against bacterial biofilms and efficacy perspectives of complementary inhalation therapy in chronic and recurrent upper respiratory tract infections. Infez Med. Jun 2013;21(2):117-24.
[14] Sadlon AE. Immune-modifying and antimicrobial effects of Eucalyptus oil and simple inhalation devices. Alternative Medicine Rev. 2010 Apr; 15(1):33-47.
[15]Juergens UR. Anti-inflammatory properties of the monoterpene 1.8-cineole: current evidence for co-medication in inflammatory airway diseases. Drug research (Stuttgart). 2014 Dec; 64(12):638-46.
[16] Annalucia Serafino. Stimulatory effect of Eucalyptus essential oil on innate cell-mediated immune response. BMC Immunology 2008, 9:17.
[17]Lazzarini E., Lonardoni A. Manuale pratico di fitoterapia. Ricette, preparazione e uso delle piante medicinali. Edizioni Mediterranee.
[18]Assessment report on Grindelia robusta Nutt., Grindelia squarrosa (Pursh) Dunal, Grindelia humilis Hook. et Arn., Grindelia camporum Greene, herba EMA/HMPC/748218/2011© European Medicines Agency, 2013.
[19] BHP 1976, Duke 1985; ESCOP 2009.
[20]Ministero della Salute Decreto 9 luglio 2012 - Disciplina dell'impiego negli Integratori Alimentari di sostanze e preparati vegetali. Linee Guida Ministeriali di Riferimento per gli effetti fisiologici.
[21] Slawomira Nowak, et al. Phenolic acids in the flowers and leaves of Grindelia Robusta Nutt. And Grindelia Squarrosa Dun. (Asteraceae). Acta Poloniae Pharmaceutica and Drug Research, Vol. 69 No. 4 pp. 693-698, 2012.
[22] La VD, et al. Active principles of Grindelia robusta exert anti-inflammatory properties in a macrophage model. Phytotherapy Research. 2010 Nov; 24(11):1687-92.
[23]Blazevic I. et al. Hedge mustard (Sisymbrium officinale): chemical diversity of volatiles and their antimicrobial activity. Chemistry & Biodiversity. 2010 Aug; 7(8):2023-34.
[24] Di Sotto A, et al. Pharmacological and phytochemical study on a Sisymbrium officinale Scop. extract. Journal of Ethnopharmacology. 2010 Feb 17;127(3):731-6.
[25] Eva Arnold et al. Inhibition of Cytosolic Phospholipase A2 (cPLA2) by Medicinal Plants in Relation to Their Phenolic Content. Molecules 2015, 20, 15033-15048.
[26] Taylor JL. An analysis of polyphenolic blackcurrant (Ribes Nigrum) extracts for the potential to modulate allergic airway inflammation. Master of Science in Nutritional Science at Massey University, New Zeland. 2009.
[27] Brigo B. Oligoelementi e litoterapici nella pratica clinica. Milano. Ed. Tecniche Nuove.
[28] Lei Liu, et al. The antiadenovirus Activities of Cinnamaldehyde In Vitro. Science. November 2009. Volume 40 Numer 11.
[29] Selvarani Vimalanathan et al. Anti-influenza virus activity of essential oils and vapors. American Journal of Essential Oils and Natural Products. August 2014

## Claims

1. Composition suited to be nebulised through aerosol therapy and to be used as a coadjuvant in the treatment of colds and diseases of the respiratory tract in general, comprising cinnamon-based phytocomplexes
**characterized in that** it comprises:
- water;
- copper gluconate;
- polysorbate 80;
- cinnamon essential oil;
- thyme essential oil;
- grindelia fluid extract;
- erysimum fluid extract;
- glycerine macerate of blackcurrant (ribes nigrum)
and a further ingredient selected between eucalyptus essential oil and mountain pine essential oil.

2. Composition suited to be administered to adult patients as a coadjuvant in the context of a method for the treatment of colds and diseases of the respiratory tract in general according to claim 1, **characterized in that** it comprises eucalyptus essential oil.

3. Composition suited to be administered to adult patients as a coadjuvant in the context of a method for the treatment of colds and diseases of the respiratory tract in general according to claim 2, **characterized in that** it comprises:
| | |
|---|---|
| - sea water | 0.001-100 % |
| - copper gluconate | 0.001-0.1 % |
| - polysorbate 80 | 0.1-0.5 % |
| - cinnamon essential oil | 0.001-0.1 % |
| - thyme essential oil | 0.001-0.1 % |
| - eucalyptus essential oil | 0.001-0.1 % |
| - grindelia fluid extract | 0.001-0.1 % |
| - erysimum fluid extract | 0.001-0.1 % |
| - glycerine macerate of blackcurrant | 0.001-0.1 %. |

4. Composition suited to be administered to paediatric patients as a coadjuvant in the context of a method for the treatment of colds and diseases of the respiratory tract in general according to claim 1, **characterized in that** it comprises mountain pine essential oil, excluding eucalyptus essential oil from the formula.

5. Composition suited to be administered to paediatric patients as a coadjuvant in the context of a method for the treatment of colds and diseases of the respiratory tract in general according to claim 4, **characterized in that** it comprises:
| | |
|---|---|
| - sea water | 0.001-100 % |
| - copper gluconate | 0.001-0.1 % |
| - polysorbate 80 | 0.1-0.5 % |
| - cinnamon essential oil | 0.001-0.1 % |
| - thyme essential oil | 0.001-0.1 % |
| - mountain pine essential oil | 0.001-0.1 % |
| - grindelia fluid extract | 0.001-0.1 % |
| - erysimum fluid extract | 0.001-0.1 % |
| - glycerine macerate of blackcurrant | 0.001-0.1 %. |

6. Composition according to the preceding claims, **characterized in that** said water is sea water.

## Patentansprüche

1. Zusammensetzung, die zum Vernebeln bei einer Aerosoltherapie und zum Verwenden als Adjuvans bei der Behandlung von Erkältungen und Erkrankungen der Atemwege im Allgemeinen geeignet ist, umfassend Phytokomplexe auf Zimtbasis,
**dadurch gekennzeichnet, dass** sie Folgendes umfasst:
- Wasser;
- Kupfergluconat;
- Polysorbat 80;
- ätherisches Zimtöl;
- ätherisches Thymianöl;
- Grindelia-Fluidextrakt;
- Erysimum-Fluidextrakt;
- Ribes Nigrum Glycerinmazerat
und einen weiteren Inhaltsstoff, ausgewählt aus ätherischem Eukalyptusöl und ätherischem Bergkieferöl.

2. Zusammensetzung nach Patentanspruch 1, die zum Verabreichen an erwachsene Patienten als Adjuvans im Rahmen eines Verfahrens zur Behandlung von Erkältungen und Erkrankungen der Atemwege im Allgemeinen geeignet ist, **dadurch gekennzeichnet, dass** sie ätherisches Eukalyptusöl umfasst.

3. Zusammensetzung nach Patentanspruch 2, die zum Verabreichen an erwachsene Patienten als Adjuvans im Rahmen eines Verfahrens zur Behandlung von Erkältungen und Erkrankungen der Atemwege im Allgemeinen geeignet ist, **dadurch gekennzeichnet, dass** sie Folgendes umfasst:
| | |
|---|---|
| - Meerwasser | 0,001-100 % |
| - Kupfergluconat | 0,001-0,1 % |
| - Polysorbat 80 | 0,1-0,5 % |
| - ätherisches Zimtöl | 0,001-0,1 % |
| - ätherisches Thymianöl | 0,001-0,1 % |
| - ätherisches Eukalyptusöl | 0,001-0,1 % |
| - Grindelia-Fluidextrakt | 0,001-0,1 % |
| - Erysimum-Fluidextrakt | 0,001-0,1 % |
| - Ribes Nigrum Glycerinmazerat | 0,001-0,1 %. |

4. Zusammensetzung nach Patentanspruch 1, die zum Verabreichen an pädiatrische Patienten als Adjuvans im Rahmen eines Verfahrens zur Behandlung von Erkältungen und Erkrankungen der Atemwege im Allgemeinen geeignet ist, **dadurch gekennzeichnet, dass** sie ätherisches Bergkieferöl umfasst und ätherisches Eukalyptusöl aus der Formulierung ausgeschlossen ist.

5. Zusammensetzung nach Patentanspruch 4, die zum Verabreichen an pädiatrische Patienten als Adjuvans im Rahmen eines Verfahrens zur Behandlung von Erkältungen und Erkrankungen der Atemwege im Allgemeinen geeignet ist, **dadurch gekennzeichnet, dass** sie Folgendes umfasst:
| | |
|---|---|
| - Meerwasser | 0,001-100 % |
| - Kupfergluconat | 0,001-0,1 % |
| - Polysorbat 80 | 0,1-0,5 % |
| - ätherisches Zimtöl | 0,001-0,1 % |
| - ätherisches Thymianöl | 0,001-0,1 % |
| - ätherisches Bergkieferöl | 0,001-0,1 % |
| - Grindelia-Fluidextrakt | 0,001-0,1 % |
| - Erysimum-Fluidextrakt | 0,001-0,1 % |
| - Ribes Nigrum Glycerinmazerat | 0,001-0,1 %. |

6. Zusammensetzung nach den vorhergehenden Patentansprüchen, **dadurch gekennzeichnet, dass** das besagte Wasser Meerwasser ist.

## Revendications

1. Composition apte à être nébulisée par aérosolthérapie et à être utilisée en tant que coadjuvant dans le traitement des rhumes et des maladies des voies respiratoires en général, comprenant des phytocomplexes à base de cannelle
**caractérisée en ce qu'**elle comprend :
- - de l'eau ;
- - du gluconate de cuivre ;
- - du polysorbate 80 ;
- - de l'huile essentielle de cannelle ;
- - de l'huile essentielle de thym ;
- - de l'extrait fluide de grindelia ;
- - de l'extrait fluide d'erysimum ;
- - du macérat glycériné de cassis (ribes nigrum)
et un autre ingrédient choisi entre l'huile essentielle d'eucalyptus et l'huile essentielle de pin de montagne.

2. Composition apte à être administrée à des patients adultes en tant que coadjuvant dans le cadre d'une méthode de traitement des rhumes et des maladies des voies respiratoires en général selon la revendication 1, **caractérisée en ce qu'**elle comprend de l'huile essentielle d'eucalyptus.

3. Composition apte à être administrée à des patients adultes en tant que coadjuvant dans le cadre d'une méthode de traitement des rhumes et des maladies des voies respiratoires en général selon la revendication 2, **caractérisée en ce qu'**elle comprend :
| | |
|---|---|
| - - de l'eau de mer | 0,001-100 % |
| - - du gluconate de cuivre | 0,001-0,1 % |
| - - du polysorbate 80 | 0,1-0,5 % |
| - - de l'huile essentielle de cannelle | 0,001-0,1 % |
| - - de l'huile essentielle de thym | 0,001-0,1 % |
| - - de l'huile essentielle d'eucalyptus | 0,001-0,1 % |
| - - de l'extrait fluide de grindelia | 0,001-0,1 % |
| - - de l'extrait fluide d'erysimum | 0,001-0,1 % |
| - du macérat glycériné de cassis | 0,001-0,1 %. |

4. Composition apte à être administrée à des patients pédiatriques en tant que coadjuvant dans le cadre d'une méthode de traitement des rhumes et des maladies des voies respiratoires en général selon la revendication 1, **caractérisée en ce qu'**elle comprend de l'huile essentielle de pin de montagne, à l'exclusion de l'huile essentielle d'eucalyptus de la formule.

5. Composition apte à être administrée à des patients pédiatriques en tant que coadjuvant dans le cadre d'une méthode de traitement des rhumes et des maladies des voies respiratoires en général selon la revendication 4, **caractérisée en ce qu'**elle comprend :
| | |
|---|---|
| - - de l'eau de mer | 0,001-100 % |
| - - du gluconate de cuivre | 0,001-0,1 % |
| - - du polysorbate 80 | 0,1-0,5 % |
| - - de l'huile essentielle de cannelle | 0,001-0,1 % |
| - - de l'huile essentielle de thym | 0,001-0,1 % |
| - - de l'huile essentielle de pin de montagne | 0,001-0,1 % |
| - - de l'extrait fluide de grindelia | 0,001-0,1 % |
| - - de l'extrait fluide d'erysimum | 0,001-0,1 % |
| - - du macérat glycériné de cassis | 0,001-0,1 %. |

6. Composition selon les revendications précédentes, **caractérisée en ce que** ladite eau est de l'eau de mer.
